(19) Europäisches Patentamt
European Patent Office
Office européen des brevets

(11) Publication number: 0 170 623 B1

(12) EUROPEAN PATENT SPECIFICATION

(45) Date of publication of patent specification: 28.11.90

(51) Int. Cl.[5]: A 61 K 37/02 // C07K7/06

(21) Application number: 85810342.7

(22) Date of filing: 24.07.85

(60) Divisional application 89121379.5 filed on 24/07/85.

# (54) Novel pharmaceutical use of (NVA)2-cyclosporine.

(30) Priority: 02.08.84 GB 8419716
02.08.84 GB 8419715

(43) Date of publication of application:
05.02.86 Bulletin 86/06

(45) Publication of the grant of the patent:
28.11.90 Bulletin 90/48

(84) Designated Contracting States:
AT BE CH DE FR GB IT LI LU NL SE

(56) References cited:
GB-A-2 033 398
US-A-4 220 641

PROG. CLIN. BIOL. RES. vol. 146, July 1984, pages 415-421; A.R. Liss Inc., New York, US;

CHEMICAL ABSTRACTS, vol. 97, no. 11, September 13, 1982, page 58, ref. no. 84951z; Columbus, Ohio, US

CHEMICAL ABSTRACTS, vol. 94, no. 23, June 8, 1981, page 47, ref. no. 185269u; Columbus, Ohio, US

IMMUNOLOGY, vol. 55, no. 2, juin 1985, pages 249-255

(73) Proprietor: SANDOZ AG
Lichtstrasse 35
CH-4002 Basel (CH)
(84) BE CH FR GB IT LI LU NL SE

(73) Proprietor: SANDOZ-PATENT-GMBH
Humboldtstrasse 3
D-7850 Lörrach (DE)
(84) DE

(73) Proprietor: SANDOZ-ERFINDUNGEN
Verwaltungsgesellschaft m.b.H.
Brunner Strasse 59
A-1235 Wien (AT)
(84) AT

(72) Inventor: Borel, Jean-François
Dornacherweg 4
CH-4144 Arlesheim (CH)
Inventor: Donatsch, Peter
Herrenweg 34
CH-4123 Allschwil (CH)
Inventor: Hiestand, Peter
Schönenbuchstrasse 13A
CH-4123 Allschwil (CH)
Inventor: Ryffel, Bernhard
Schweizergasse 8
CH-4054 Basel (CH)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 170 623 B1

Courier Press, Leamington Spa, England.

## Description

The present invention relates to a new use, in particular a new pharmaceutical use, for the compound cyclosporin G, also known in accordance with current nomenclature and referred to throughout the present specification and claims as (Nva)[2]-Cyclosporine, especially to its use in the preparation of pharmaceutical compositions for application in relation to said use.

(Nva)[2]-Cyclosporin, which has the formula I

-A-B-Sar-MeLeu-Val-MeLeu-Ala-(D)Ala-MeLeu-MeLeu-MeVal- (I)

wherein A represents the residue of formula II

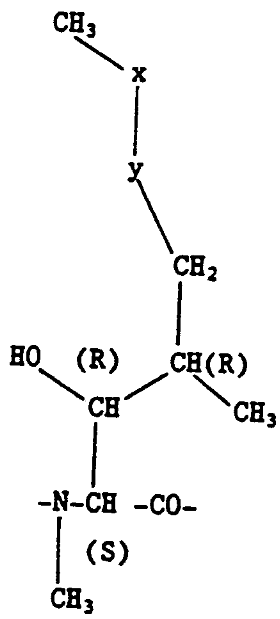

(II)

in which —x—y— is —CH=CH-(trans), and B is Nva, is known and is described together with processes for its production as well as various pharmaceutical utilities, e.g. in US patent no. 4,288,431.

Chemically (Nva)[2]-Cyclosporine belongs to the distinct and now substantial class of natural and synthetic or semi-synthetic undecapeptides collectively designated as the cyclosporins [see for example US patent No. 4,117,118 (cyclosporin A or Cyclosporine); US patents Nos. 4,108,985 and 4,210,581 (cyclosporin C or (Thr)[2]-Cyclosporine, and derivatives); as well as European patent publication No. 0,058,134 B1 and Helv. Chim. Acta. 65, Fasc. 5, pp. 1655—1677 (1982) disclosing yet further naturally occurring and synthetic or semi-synthetic cyclosporins including inter al. (Leu)[10]-Cyclosporine, [(D)Ser][8]-Cyclosporine and (Val)[11]-Cyclosporine].

Of the cyclosporins the parent compound, cyclosporin A or Cyclosporine, has so far received the most attention. As disclosed in the aforementioned US patent No. 4,117,118 it possesses anti-inflammatory and anti-arthritic properties, as well as immunosuppressive properties which render the compound of especial utility in the prevention of rejection following organ transplant operations as well as in the treatment of auto-immune diseases.

The primary area of clinical investigation of Cyclosporine has been in its application to recipients of organ, e.g. heart, lung, combined heart-lung, liver, kidney, pancreatic, bone-marrow, skin and corneal transplants and, in particular, allogenic organ transplants. Cyclosporine is now commercially available for use in relation to organ transplant and a measure of its remarkable success is provided by the uncommonly wide attention the compound has received, not only in the scientific literature, but also in the lay press world-wide. At the same time, investigation of the applicability of Cyclosporine to the treatment of various auto-immune diseases has been intensive and reports of results in-vitro, in animal models and in clinical trials are wide-spread in the literature. The variety of auto-immune diseases is in fact very large. Though inevitably reported success for Cyclosporine has tended to vary in degree, e.g. from one auto-immune disease to another, broad utility is established and in some instances available results are sufficiently encouraging to warrant on-going world-wide specialist attention.

Specific auto-immune diseases for which Cyclosporine treatment has been investigated or proposed include multiple sclerosis, Guillain-Barré syndrome, uveitis, myesthenia gravis, Heymann nephritis, Grave's disease, Hashimoto's thyroiditis, juvenile diabetes type I, systemic lupus erythematodes, aplastic anaemia, pure red cell anaemia, idiopathic thrombocyctopaenia, polychondritis, sclerodoma, Wegner granulamatosis, dermatomyositis, chronic active hepatitis, autoimmune male infertility, psoriasis and psoriatic arthritis, Steven-Johnson syndrome, idiopathic sprue, Chron's disease, sarcoidosis, glomerulonephritis, interstititial lung fibrosis and primary billiary cirrhosis.

As disclosed e.g. in US patent specification No. 4,288,431, $(Nva)^2$-Cyclosporine also possesses anti-inflammatory and anti-arthritic properties as well as immunosuppressive properties in test methods previously applied to Cyclosporine and to have the same utility profile and clinical utility as Cyclosporine. $(Nva)^2$-Cyclosporine like Cylclosporine may thus in its turn be anticipated to find application primarily in the prevention of transplant rejection as well as in the treatment of auto-immune disease. The present invention resides in the finding that, while $(Nva)^2$-Cylclosporine may be confirmed to possess the same general utility as Cyclosporine, i.e. in terms of its applicability to prevention of organ transplant rejection and to the treatment of auto-immune diseases as a broad class, its particular profile of activity, especially its particular profile in relation to immunosuppressive effect, renders the compound surprisingly and unexpectedly advantageous in its application to uveitis.

More especially it may be shown that in relation to the above specified auto-immune disease, $(Nva)^2$-Cyclosporine exhibits particular and unexpected advantage in terms of e.g. level of activity and/or reduction or absence of undesirable side reactions in comparison with previously proposed therapy, e.g. employing Cyclosporine.

The particular and advantageous utility of $(Nva)^2$-Cyclosporine in the treatment of Uveitis can be demonstrated in recognised standard animal models as well as in clinical trials conducted for example as follows:

1. UVEITIS

Modulation of Experimental Autoimmune Uveitis (EAV)

Testing is carried out in accordance with the general methodology described by Nussenblatt et al. in Arch. Ophthal. 100, 1146—1149 (1982). Groups of 6 to 10 Lewis (♀) rats weighing ca. 150—200 g are immunized with 30 µg of bovine S antigen emulsified (1.1 p.p.w.) in complete Freund's adjuvant enriched with 2.5 mg/ml Mycrobacterium tuberculosis H 37 RA, by injection into the hind foot pad.

Test substance, i.e. $(Nva)^2$-Cyclosporine, is administered at a dosage of from 25 to 50 mg/kg/day i.m. administered on 7 consecutive days commencing 7 days after immunization. Control groups receive olive oil in place of test substance. The rats are killed on the 14th day following immunization and the eyes are removed immediately, fixed in formaldehyde, embedded in paraffin wax and stained with heamatoxylin-eosin and PAS. Histopathologic evaluation is performed in masked fashion and inflammation graded on a scale of from 0 (no inflammation) to 4 (panophthalmitis). Selected cases are examined by transmission and scanning electron microscopy. Eyes from animals exhibiting EAV show generalized inflammation of the retina and choroid with inflammatory cells enmessed in a fibrinous exudate occurring in the vitrious cavity, subretinal space and anterior chamber.

On administration of $(Nva)^2$-Cyclosporine at dosages indicated above, substantial reduction in the number of animals evidencing EAV is observed compared with results for control groups.

As previously indicated effectiveness of $(Nva)^2$-Cyclosporine in accordance with the present invention may also be demonstrated in clinical trials.

In accordance with the foregoing the present invention provides: The use of $(Nva)^2$-Cyclosporine for the manufacture of a pharmaceutical composition for the treatment of uveitis.

By "treatment" as used in the above definitions is to be understood both curative treatment as well as prophylactic treatment where appropriate, e.g. as indicated in the above described animal model test methods.

Doses for clinical use in accordance with the method of the invention will of course vary depending upon, e.g. the mode of administration, the specific condition of the subject to be treated and the effect desired. In addition dosaging will generally require adjustment for individual patients receiving treatment in order to establish an appropriate long-term drug serum concentration, e.g. by administration of an initial daily starting or "loading" dose with subsequent dose adjustment (generally reduction) in accordance with achieved serum levels as determined, e.g. by regular RIA monitoring. However in general satisfactory results are obtained on administration of $(Nva)^2$-Cyclosporine in a dose range of from 3 to 30, e.g. from 10 to 25 mg/kg body weight/day administered to the patient orally once or, in divided doses, 2 or 3× a day. Where i.v. administration is indicated, for example in the initial phase of treatment, lower dosages, e.g. of the order from 1 to 10, e.g. ca. 3 to 5 mg/kg/day are generally indicated. A suitable oral daily dosage, e.g. for adult patients, is accordingly of the order of from 225 to 2,000, e.g. from 750 to 1,800 mg/day, and unit dosage forms for oral administration suitably comprise from 75 to 2,000, e.g. from 250 to 1,500 mg $(Nva)^2$-Cyclosporine/dose, together with a pharmaceutically acceptable diluent or carrier therefor.

$(Nva)^2$-Cyclosporine is tolerated at dosages contemplated for use in accordance with the present invention. Indeed it has very surprisingly been found that while $(Nva)^2$-Cyclosporine generally possesses a high order of immunosuppressive activity, and may be shown to possess particularly advantageous

properties in relation to immunosuppressive use in accordance with the present invention, e.g. as hereinbefore indicated, it is unexpectedly lacking in side effects, in particular hepato- and nephro-toxic effects, hitherto experienced employing Cyclosporine as medication. This relative freedom from undesirable side effects may for example be demonstrated in standard short-term toxicity trials.

One such trial is carried out employing 3 groups of 5 HAN Wistar rats. Group 1 receives 100 mg/kg $(Nva)^2$-Cyclosporine/day, Group 2 receives 100 mg/kg Cyclosporine/day and Group 3 (control) remains untreated. Both $(Nva)^2$-Cyclosporine and Cyclosporine are administered in solution in olive oil by means of a stomach tube. The following parameters are evaluated: body weight; mortality; serum and urine analysis; kidney and liver analysis, macroscopy and histology (urine volume, kidney weight, creatine clearance, Na and K levels, SGPT and SGOT levels etc); an neurological symptoms. Results obtained for neurological symptoms, mortality, kidney biochemistry and histoloty (in particular swelling/regeneration of tubular epithelial cells and tubular necrosis) and liver biochemistry in Group 1 are in all cases directly comparable with results obtained for control Group 3 and show over all marked superiority in comparison with results obtained for Group 2. No adverse reaction in Group 1 as compared with Group 2 is observed in respect of other parameters measured.

Suitable galenic formulations for the administration of cyclosporins, including the current commercially available Cyclosporine drink solution, are described and claimed in DOS 29 07 460 (= Japanese patent application No. 27228/1979, U.K. patent specification No. 2 015 339 B and US patent No. 4,388,307). Pharmaceutical compositions for use in accordance with the present invention may be prepared directly analogously to the methods disclosed therein, employing $(Nva)^2$-Cyclosporine as active ingredient. Liquid oral compositions comprising $(Nva)^2$--Cyclosporine (e.g. drink solutions), as the Cyclosporine drink solution itself, are suitably administered together with a chocolate flavouring agent, e.g. as described in Example 1 of the aforementioned US patent No. 4,388,307.

The following example is for the preparation of liquid pharmaceutical preparations suitable for the administration of $(Nva)^2$-Cyclosporine.

All percentages are by weight.

Example 1
$(Nva)^2$-Cyclosporine Drink Solution

| | Component | | Content mg/ml |
|---|---|---|---|
| i) | $(Nva)^2$-Cyclosporine | | 100.0 |
| ii) | Ethanol (absolute) | | 150.0 |
| iii) | Labrafil® 2125 | | 350.0 |
| iv) | Corn oil | to a total of | 922.0 (≡1 ml) |

The desired quantity of (i) is dissolved in components (ii) and (iv) in conventional manner, the solution brought to an end volume of 100% by addition of (iii) and the obtained solution filled into individual containers in an amount giving the required dosage of i). The solution is preferably ingested undiluted.

**Claim**

The use of $(Nva)^2$-Cyclosporine for the manufacture of a pharmaceutical composition for the treatment of uveitis.

**Patentanspruch**

Die Anwendung von $(Nva)^2$-Cyclosporine zur Herstellung einer pharmazeutischen Zusammensetzung zur Behandlung von Uveitis.

**Revendication**

L'utilisation de la $(Nva)^2$-Cyclosporine pour la préparation d'une composition pharmaceutique pour le traitement de l'uvéite.